# EUROPÄISCHE PATENTSCHRIFT

(11) **EP 3 528 273 B1**
(45) Veröffentlichungstag und Bekanntmachung des Hinweises auf die Patenterteilung: **23.08.2023**
(21) Anmeldenummer: 18157701.6
(22) Anmeldetag: 20.02.2018
(51) Int. Cl.: H01J 33/04, A23B 9/06, G21K 5/08, B65B 55/08, A61L 2/08, A23L 3/26

(54) **VORRICHTUNG UND VERFAHREN ZUM PASTEURISIEREN UND/ODER STERILISIEREN VON PARTIKELFÖRMIGEM GUT**
DEVICE AND METHOD FOR PASTEURISING AND/OR STERILISING PARTICULATE MATERIAL
DISPOSITIFS ET PROCÉDÉ DE PASTEURISATION ET/OU DE STÉRILISATION DE PRODUITS PARTICULAIRES

(43) Veröffentlichungstag der Anmeldung: 21.08.2019
(73) Patentinhaber: Bühler AG, 9240 Uzwil (CH)
(72) Erfinder: CURRIE, Alasdair, London N7 8QB (GB)
(74) Vertreter: Hepp Wenger Ryffel AG

(56) Entgegenhaltungen:
- DE-A1- 19 518 623
- JP-A- H11 133 193
- JP-A- 2000 304 900
- JP-A- 2002 078 472
- JP-A- 2002 085 029
- JP-A- 2007 010 450
- JP-B2- 3 569 329
- JP-B2- 3 777 080

## Beschreibung

Die vorliegende Erfindung betrifft Vorrichtungen und Verfahren zum Pasteurisieren und/oder Sterilisieren von partikelförmigem Gut mit Hilfe eines Elektronenstrahls.

Als partikelförmig werden hier und im Folgenden unter anderem aus Körnern und/oder Flocken bestehende Güter bezeichnet, wobei die Partikel beispielsweise eine kugelförmige, plattenförmige oder kantige Form haben können. Es kann sich auch um gemahlene Partikel handeln. Durch die Pasteurisierung und/oder Sterilisierung können beispielsweise Mikroorganismen zumindest grösstenteils abgetötet oder unschädlich gemacht werden. Insbesondere kann eine Reduktion von schädigenden Mikroorganismen um mindestens eine, bevorzugt mindestens fünf, besonders bevorzugt mindestens sieben Grössenordnungen erreicht werden.

Eine gattungsgemässe Vorrichtung ist beispielsweise aus der EP 1 080 623 B1 bekannt. Diese Vorrichtung enthält Vibrationsförderer, mit denen Saatgut zu einem transparenten Vorhang vereinzelt werden kann. Dieser Vorhang wird dann durch ein Elektronenfeld geführt, das von einem Elektronenbeschleuniger erzeugt wird und beispielsweise eine Sterilisierung des Saatguts bewirken kann. Um das Saatgut von einem Austrittsfenster des Elektronenbeschleunigers fernzuhalten, wird ein Gitter eingesetzt.

Aus der US 5,801,387 A ist eine weitere gattungsgemässe Vorrichtung bekannt. In der dortigen erfindungsgemässen Ausführung wird ein partikelförmiges Gut mit einem Vibrationsförderer in einen horizontalen Luftstrom eindosiert und dann einem Elektronenstrahl ausgesetzt. Die Behandlungszone ist gegenüber dem Elektronestrahlquelle mit einem Fester abgetrennt.

Aus der EP 0 705 531 B1 ist eine weitere Vorrichtung bekannt, die das Saatgut mittels einer nicht näher beschriebenen Dosiereinrichtung in eine Prozesskammer eingeleitet wird, in der es senkrecht durch einen Elektronenstrahl fällt. Zur Kühlung des Strahlaustrittsfensters dient ein Kühlgas, das durch ein Düsensystem mit einem Einlass und einem Auslass an dem Strahlaustrittsfenster vorbeigeführt wird.

Weitere Vorrichtungen und Verfahren zum Pasteurisieren und/oder Sterilisieren von partikelförmigem Gut sind aus der internationalen Patentanmeldung PCT/EP2017/070842 der Anmelderin offenbart. Die Vorrichtungen enthalten mindestens eine Elektronenquelle zum Erzeugen eines Elektronenstrahls und eine Behandlungszone, in der das Gut mittels des Elektronenstrahls pasteurisierbar und/oder sterilisierbar ist.

Die PCT/EP2017/070842 offenbart eine Vorrichtung, die in einem Bereich der Behandlungszone einen Gutkanal aufweist, in dem das Gut mittels des Elektronenstrahls pasteurisierbar und/oder sterilisierbar ist. Die Vorrichtung weist mindestens einen von einem Fluid durchströmbaren Nebenkanal auf, welcher zumindest teilweise zwischen der Elektronenquelle und dem Gutkanal verläuft und vom Gutkanal fluidgetrennt ist. Das durch den Nebenkanal strömende Fluid kann dazu eingesetzt werden, die Elektronenquelle und insbesondere ein Austrittsfenster der Elektronenquelle zu kühlen.

Bei einer Vorbeiführung von Kühlfluid an dem Austrittsfenster der Elektronenquelle wird vor allem die das Fenster umgebende Luft gekühlt und somit indirekt auch das Austrittsfenster. Die Kühlung erfolgt also auf indirekte Weise und hängt von den Strömungsverhältnissen in der Umgebung des Austrittsfensters ab.

Die PCT/EP2017/070842 zeigt ausserdem eine Schutzfolie, die zwischen der Elektronenquelle und dem Gutkanal angeordnet ist und für den Elektronenstrahl zumindest teilweise durchlässig ist. Bevorzugt trennt diese Schutzfolie den Gutkanal vom Nebenkanal. Ebenfalls bevorzugt ist der Nebenkanal zumindest teilweise zwischen der Elektronenquelle und der Schutzfolie angeordnet.

Das sich darin befindende Fluid dient also zum Kühlen der Schutzfolie und wird aber beim Betrieb der Vorrichtung dem Elektronenstrahl ausgesetzt und kann dadurch erwärmt werden.

Weiterhin offenbart sind eine Kassette zum Einsetzen in eine Vorrichtung zum Pasteurisieren und/oder Sterilisieren von partikelförmigem Gut.

Aus der JP 2007 010450 ist eine Vorrichtung zum Sterilisieren von Waren mit Hilfe eines Elektronenstrahls bekannt. Diese Vorrichtung enthält, unter anderem, Elektronenquellen zum Erzeugen eines Elektronenstrahls, eine für den Elektronenstrahl teilweise durchlässige Titanfolie, und einen Halterahmen, der die Titanfolie hält und der einen von einem Kühlfluid durchströmbaren Hohlraum aufweist.

Es ist eine Aufgabe der vorliegenden Erfindung, die aus dem Stand der Technik bekannten Nachteile zu überwinden. Insbesondere sollen Vorrichtungen und Verfahren bereitgestellt werden, das eine verlässliche, direkte und/oder möglichst homogene Kühlung des Austrittsfensters erlaubt.

Die Aufgabe wird durch eine Vorrichtung zum Pasteurisieren und/oder Sterilisieren von partikelförmigem Gut gemäss Anspruch 1 gelöst.

Das Gut wird als "frei fallend" bezeichnet, wenn die Flugbahnen der einzelnen Partikel des Guts allein durch ihre Geschwindigkeit, die auf sie einwirkende Schwerkraft und gegebenenfalls ein Prozessgas, von dem das Gut umgeben ist, bestimmt werden. Insbesondere rutschen die Partikel des Guts nicht auf einer Fläche durch die Behandlungszone. Das Prozessgas kann beispielsweise Luft sein. Es ist jedoch auch denkbar, dass als Prozessgas ein Gas eingesetzt wird, welches eine Ozonbildung verhindert, wie beispielsweise Stickstoff.

Weiterhin ist es auch denkbar und liegt im Rahmen der Erfindung, dass die Vorrichtung mehrere Behandlungszonen aufweist. Auf diese Weise kann eine noch effektivere Pasteurisierung und/oder Sterilisierung erreicht werden. Alternativ kann das Gut mehrmals durch ein und dieselbe Behandlungszone geführt werden.

Die Vorrichtung enthält einen im Bereich der Behandlungszone angeordneten Gutkanal, in dem das Gut mittels des Elektronenstrahls pasteurisierbar und/oder sterilisierbar ist.

Mittels des Elektronenstrahls kann nicht nur das Gut selbst behandelt werden, sondern auch allfälliges das Gut umgebendes Prozessgas und/oder weitere mit dem Gut strömende Partikel wie beispielsweise Staub.

Die Vorrichtung umfasst flächige Schutzelemente, die zwischen den Elektronenquellen und dem Gutkanal angeordnet sind. Die Schutzelemente sind für den Elektronenstrahl zumindest teilweise durchlässig. Die Schutzelemente enthalten insbesondere ein Metall, bevorzugt Titan, bevorzugt bestehen sie aus Metall.

Das Schutzelement verhindert, dass das Gut zu der Elektronenquelle gelangt und schützt die Elektronenquelle, insbesondere das selbst mit einer dünnen und entsprechend empfindlichen Folie abgedeckte Austrittsfenster der Elektronenquelle.

Die Halterung ist so ausgeführt, dass zwischen Schutzelement und Halterahmen eine Verbindung besteht, die eine gute Wärmeleitfähigkeit ermöglicht. Das Schutzelement steht mit dem Halterahmen insbesondere in einem direkten Kontakt.

Das Schutzelement kann in den Halterahmen eingespannt sein, in dem Halterahmen eingeklemmt sein, auf dem Halterahmen aufgeklebt oder aufgeschweisst sein.

Bevorzugt ist der Halterahmen lösbar an der Vorrichtung angebracht, so dass für ein Austauschen des Schutzelements der Halterahmen entnehmbar und gegebenenfalls ebenfalls austauschbar ist.

Mit einer Druckmessung kann festgestellt werden, ob das Schutzelement noch intakt ist, insbesondere wenn es sich um eine Schutzfolie handelt.

Der Halterahmen umgibt das Schutzelement zumindest teilweise. Bevorzugt umgibt der Halterahmen das Schutzelement auf einem erheblichen Anteil des Umfangs derart, dass über den Umfang ein möglichst gleichmässig verteilter Wärmeaustausch ermöglicht wird.

Der Hohlraum dient zum Leiten eines Kühlfluids, also eines Kühlgases oder einer Kühlflüssigkeit am Halterahmen oder durch den Halterahmen hindurch. Der Hohlraum ist so angeordnet, dass ein guter thermischen Kontakt zwischen dem Kühlfluid und dem Halterahmen herstellbar ist.

Der Halterahmen kann so angeordnet sein, dass er sich nicht im Elektronenstrahl befindet. Das Kühlfluid wird also an dem Elektronenstrahl vorbeigeleitet und ist somit dem Elektronenstrahl nicht direkt ausgesetzt und erfährt auch keine direkte Erwärmung.

Bevorzugt ist der Halterahmen aus einem Material, das nicht oder nur wenig durchlässig für einen Elektronstrahl ist.

Als Kühlfluid kann das Prozessgas dienen.

Der Hohlraum ist bevorzugt von der Behandlungszone fluidgetrennt. Unter einer Fluidtrennung wird hier verstanden, dass weder das Fluid vom Hohlraum in den Gutkanal eintreten kann noch das Gut und allfälliges Prozessgas, welches das Gut umgibt, vom Gutkanal in den Hohlraum eintreten kann. Das Kühlfluid ist dann frei wählbar und muss nicht auf das Gut abgestimmt werden.

Der Hohlraum ist bevorzugt, bis auf mindestens einen Einlass und mindestens einen Auslass, geschlossen ausgebildet, zum Beispiel als Röhre, also als im Wesentlichen zylindrischer Hohlkörper, der vollständig von dem Halterahmen umgeben sein kann.

Der Hohlraum kann auch in einem Rohr ausgebildet sein, welches an dem Halterahmen anliegt.

Das den Halterahmen durchströmende Kühlfluid kommt bevorzugt mit dem Schutzelement nicht in Kontakt. Es kann also mit Flüssigkeit gekühlt werden und mit schnell transportiertem Kühlfluid, da nicht die Gefahr besteht, dass das Schutzelement in Schwingung versetzt wird. Schnell transportiertes Kühlfluid kann die Wärme zügig abführen.

Bevorzugt ist der Hohlraum Teil eines Kühlkreislaufs, welcher von einem Kühlfluid durchströmt wird, und in dem das Kühlfluid insbesondere wieder abkühlt.

Die Vorrichtung kann mindestens eine Pumpe umfassen, mit welcher Kühlfluid zum Schutzelement und/oder zur Elektronenquelle getrieben wird.

Der Hohlraum kann gegenüber der Umgebung teilweise offen sein. Er kann als Nut in dem Halterahmen gebildet sein.

Der Hohlraum kann beispielsweise aus Leitblechen gebildet sein, welche das Kühlfluid an den Halterahmen und/oder an dem Halterahmen vorbei leiten.

Über das Kühlfluid wird der Halterahmen temperiert. Somit wird das Schutzelement gekühlt. Die Kühlung erfolgt unabhängig von Strömungsverhältnissen. Das Kühlfluid kann in dem Hohlraum kurze Wege zurücklegen und die Kühlung erfolgt somit schnell und effektiv, wenn ein guter Wärmekontakt zwischen Halterahmen und Schutzelement sichergestellt ist.

Das Schutzelement kann als Gitter ausgebildet sein, bevorzugt ist das Schutzelement als Schutzfolie ausgebildet, die Sicherheit für die Elektronenquelle bietet und leichter zu reinigen ist.

Die Schutzfolie besteht vorzugsweise aus einem Metall, wie beispielsweise Titan, Aluminium, Gold, Silber oder Kupfer. Bei dem Metall kann es sich auch um eine Legierung handeln. In einigen Anwendungen kann die Schutzfolie beschichtet sein. Alternativ ist es auch denkbar und liegt im Rahmen der Erfindung, dass die Schutzfolie aus einem Kunststoff besteht.

Die Kühlung des Haltrahmens bewirkt zum einen eine Kühlung des Schutzelements. Gleichzeitig wird verhindert, dass der Halterahmen als Wärmespeicher dient, der dem Schutzelement wieder Wärme zuführt, wenn dieses anderweitig, zum Beispiel über einen Nebenkanal gekühlt wird.

Die Kühlung des Halterahmens dient zur Flächenkühlung des Halterahmens, der bevorzugt auf einer Temperatur unter 200°C gehalten werden sollte, um das Risiko von Staubselbstentzündungen zu vermeiden und Staubablagerungen zu verringern.

Ein gut kühlbares Schutzelement kann den das Schutzelement umgebenden Raum mitkühlen. Die Lebensdauer des Schutzelements wird durch die Kühlung verlängert.

In einer vorteilhaften Weiterbildung der Erfindung weist die Vorrichtung, wie in Anspruch 4 definiert, mindestens einen von einem Fluid durchströmbaren Nebenkanal auf, welcher zumindest teilweise zwischen Elektronenquelle und Gutkanal verläuft und vom Gutkanal fluidgetrennt ist.

Das durch den Nebenkanal strömende Fluid kann dazu eingesetzt werden, die Elektronenquelle und insbesondere ein Austrittsfenster der Elektronenquelle zu kühlen.

Alternativ oder zusätzlich kann das Fluid dafür eingesetzt werden, aufgrund des Elektronenstrahls entstehendes Ozon abzuführen. Weder diese Kühlung noch diese Abführung von Ozon aus dem Nebenkanal haben einen Einfluss auf den Fluidstrom im Gutkanal.

Unter einer Fluidtrennung wird hier verstanden, dass weder das Fluid vom Nebenkanal in den Gutkanal eintreten kann noch das Gut und allfälliges das Gut umgebendes Prozessgas vom Gutkanal in den Nebenkanal eintreten kann. Hierdurch wird eine Beschädigung oder Verschmutzung der Elektronenquelle, insbesondere eines Austrittsfensters Elektronenquelle, durch das Gut verhindert. Das Fluid kann eine Flüssigkeit oder ein Gas sein, wie beispielsweise Luft.

Der Hohlraum und der Nebenkanal können fluidgetrennt sein. Es kann jedoch auch eine Fluidverbindung bestehen, so dass durch den Nebenkanal strömendes Kühlfluid auch durch den Hohlraum strömt.

Insbesondere kann das Schutzelement den Gutkanal vom Nebenkanal trennen. Das durch den Nebenkanal strömende Fluid kommt dann in Kontakt mit dem Schutzelement und kann zu einer zusätzlichen Kühlung des Schutzelements dienen.

Alternativ oder zusätzlich kann der Nebenkanal zumindest teilweise zwischen Elektronenquelle und Schutzelement angeordnet sein. Das durch den Nebenkanal strömende Fluid kann dann einerseits die Umgebung der Elektronenquelle und damit die Elektronenquelle kühlen, zum anderen zur Kühlung des Schutzelements beitragen.

In einer vorteilhaften Weiterbildung der Vorrichtung enthält das Schutzelement, insbesondere die Schutzfolie, mindestens eine Verdickung. Bevorzugt enthält das Schutzelement mehrere Verdickungen.

Die Verdickungen erstrecken sich entlang einer Hauptebene des Schutzelements und im Wesentlichen senkrecht zu einer Gutströmungsrichtung.

Die Verdickungen stabilisieren das Schutzelement, so dass es auch Stösse von scharfkantigem Gut aushält.

Die Verdickungen können ausserdem dazu dienen, das Behandlungsfenster zu definieren, also genauer den Bereich der Behandlungszone festzulegen, in dem das Gut mittels des Elektronenstrahls pasteurisiert und/oder sterilisiert wird.

Eine Schutzfolie mit Verdickungen vereint die Vorteile einer Folie und eines Gitters.

Die Vorrichtung kann separate Kühlkreisläufe für die Elektronenquelle und das Schutzelement umfassen, die jeweils eine Kühlvorrichtung zum Abkühlen des Kühlfluids aufweisen.

Vorteilhafterweise enthält die Vorrichtung eine Kühlvorrichtung mit zwei miteinander verbundenen Kühlkreisläufen, wobei ein Kühlfluid mittels eines ersten Kühlkreislaufs dem Halterahmen zuführbar ist und mittels eines zweiten Kühlkreislaufs der Elektronenquelle zuführbar ist.

Eine besonders gute Kühlung lässt sich erreichen, wenn die Vorrichtung mindestens ein auf das Schutzelement gerichtetes Gebläse zur weiteren Kühlung des Schutzelements enthält.

Das durch das Gebläse bewegte Kühlgas kann direkt von dem Gebläse auf das Schutzelement geführt werden oder beispielsweise durch den Nebenkanal zum Schutzelement geleitet werden.

Mit besonderem Vorteil enthält die Vorrichtung eine Kassettenaufnahme zum Aufnehmen einer Kassette, wobei die Kassette zumindest teilweise den Gutkanal und insbesondere den mindestens einen Nebenkanal begrenzt. Die Kassette kann die Halterahmen oder eine Aufnahme für die Haltrahmen enthalten. Die Halterahmen können lösbar in der Kassette aufgenommen oder aufnehmbar sein.

Bevorzugt sind Halterahmen für zwei Schutzelemente integraler Bestandteil der Kassette und/oder einteilig mit der Kassette ausgebildet.

Besonders vorteilhaft enthält die Kassette den kompletten Gutkanal und umfasst insbesondere Halterahmen für zwei gegenüber liegende Schutzelemente. Die gesamte Behandlungszone kann zum Beispiel zu Wartungs- oder Reinigungszwecken entfernt und/oder ausgetauscht werden.

Die Kassette umfasst bevorzugt den Hohlraum, der als durchgehende Röhre ausgebildet ist und zur Kühlung beider Schutzelemente und der Kassette dient.

In der Kassette kann eine Druckmessvorrichtung angeordnet sein, über die feststellbar ist, ob das Schutzelement noch intakt ist.

Weiterhin vorteilhaft sind die Elektronenquellen derart beweglich, insbesondere schwenkbar und/oder verschiebbar, relativ zur Kassettenaufnahme angeordnet, dass sie von der Kassette weg bewegbar sind. Dies vereinfacht den Zugang zur Kassette und damit auch zum Schutzelement. Das Schutzelement kann folglich leichter ausgetauscht werden, wenn es vom Gut verschmutzt oder beschädigt wurde. Dazu werden die Schutzelemente beispielsweise zusammen mit dem Halterahmen aus der Kassette entfernt.

Die Kassette ist bevorzugt in eine Kassettenaufnahme der Vorrichtung einschiebbar in einer Richtung quer zur Gutströmungsrichtung. Die Kassettenaufnahme der Vorrichtung kann dafür einen Rahmen mit Führung umfassen, in welcher die Kassette gleitet und in welcher sich Dichtungen befinden.

Die Kassette besitzt vorteilhafterweise Anschlussstutzen zum Anschliessen des Hohlraums an einen Kühlkreislauf.

Die Kassette ist vorteilhafterweise mit Fluidsteckern zum Ankoppeln an einen Kühlkreislauf ausgestattet.

Die Kassette enthält Begrenzungsflächen zum zumindest teilweisen Begrenzen eines Gutkanals und insbesondere mindestens eines Nebenkanals der Vorrichtung. Die Kassette enthält Haltrahmen für Schutzelemente oder eine Aufnahme für Halterahmen. Die Kassette ist bevorzugt so ausgeführt wie oben beschrieben.

Die Kassette ist derart in die Kassettenaufnahme einsetzbar, dass die Vorrichtung im Bereich einer Behandlungszone einen Gutkanal aufweist, in dem das Gut mittels des Elektronenstrahls pasteurisierbar und/oder sterilisierbar ist, und dass die Vorrichtung insbesondere mindestens einen von einem Fluid durchströmbaren Nebenkanal aufweist, welcher zumindest teilweise zwischen Elektronenquelle und Gutkanal verläuft und vom Gutkanal fluidgetrennt ist. Die Begrenzungsflächen der Kassette begrenzen dann zumindest teilweise den Gutkanal und insbesondere den mindestens einen Nebenkanal.

Dabei strömt das Gut im Bereich der Behandlungszone durch einen Gutkanal, in dem es mittels des Elektronenstrahls pasteurisiert und/oder sterilisiert wird.

Gemäss diesem Aspekt strömt ein Fluid durch den Hohlraum und insbesondere durch mindestens einen Nebenkanal, welcher zumindest teilweise zwischen der Elektronenquelle und dem Gutkanal verläuft und vom Gutkanal fluidgetrennt ist. Wie bereits ausgeführt wurde, kann mit diesem Fluid, welches eine Flüssigkeit oder ein Gas sein kann, Ozon abgeführt werden, welches auf Grund des Elektronenstrahls entstanden ist.

Alternativ oder zusätzlich kann das Fluid auch zum Kühlen der Elektronenquelle, insbesondere eines Austrittsfensters, eingesetzt werden, zum Beispiel in einem verbundenen Kühlkreislauf oder bei der Durchströmung eines Nebenkanals.

Das Fluid kann den Nebenkanal parallel oder entgegengesetzt zur Strömungsrichtung des Guts durchströmen. Als Fluid kann ein Gas eingesetzt wird, welches eine Ozonbildung verhindert, wie beispielsweise Stickstoff.

Das durch den Nebenkanal und/oder durch den Hohlraum strömende Fluid kann mit dem im Gutkanal strömenden Prozessgas identisch sein oder davon verschieden sein. Andere Strömungsrichtungen des Fluids sind jedoch ebenso denkbar und liegen im Rahmen der Erfindung.

Im Rahmen der vorliegenden Anmeldung beziehen sich die Begriffe "stromabwärts" und "stromaufwärts" auf die Strömungsrichtung des partikelförmigen Guts bei bestimmungsgemässer Bedienung der Vorrichtung. Folglich wird eine erste Einheit als stromabwärts von einer zweiten Einheit bezeichnet, wenn sie bei bestimmungsgemässer Bedienung der Vorrichtung vom Gut nach der zweiten Einheit passiert wird. Analog wird eine erste Einheit als stromaufwärts von einer zweiten Einheit bezeichnet, wenn sie bei bestimmungsgemässer Bedienung der Vorrichtung vom Gut vor der zweiten Einheit passiert wird.

Die Vorrichtung kann zudem eines oder mehrere der Merkmale aufweisen, die in der eingangs bereits erwähnten internationalen Patentanmeldung PCT/EP2017/070842 der Anmelderin offenbart sind, insbesondere
i) eine bevorzugt im Wesentlichen horizontal ausgerichtete erste Vibrationsfläche, welche zu Vibrationen anregbar ist, um das Gut zu fördern und zu vereinzeln,
   wobei die Behandlungszone, in der das Gut insbesondere frei fallend mittels der Elektronenstrahlen pasteurisierbar und/oder sterilisierbar ist, stromabwärts von der ersten Vibrationsfläche angeordnet ist,
   wobei die erste Vibrationsfläche eine Vielzahl von Rinnen aufweist, in denen das Gut förderbar und mittels deren es vereinzelbar ist.
ii) Zusätzlich zu i) kann die Vorrichtung stromabwärts von der ersten Vibrationsfläche und stromaufwärts von der Behandlungszone eine geneigte Rutschfläche aufweisen, welche derart ausgebildet und angeordnet ist, dass das Gut darauf in Richtung der Behandlungszone rutschen kann.
iii) Zusätzlich zu ii) kann die Rutschfläche mindestens eine Rinne, bevorzugt eine Vielzahl von Rinnen aufweisen, welche derart ausgebildet und angeordnet ist/sind, dass das Gut darin rutschen und vereinzelt werden kann.
iv) Zusätzlich zu ii) oder iii) kann die Rutschfläche bezüglich einer Horizontalen unter einem Winkel nach unten geneigt sein, der im Bereich von 45° bis 85°, bevorzugt von 55° bis 75°, besonders bevorzugt von 60° bis 70° liegt.
v) Zusätzlich zu einem der Merkmale i) bis iv) kann die Vorrichtung stromabwärts von der ersten Vibrationsfläche und stromaufwärts von der Behandlungszone, insbesondere stromaufwärts von der Rutschfläche, eine Umlenkfläche aufweisen, welche derart ausgebildet und angeordnet ist, dass das Gut darauf umgelenkt und von der ersten Vibrationsfläche zur Rutschfläche und/oder in Richtung der Behandlungszone rutschen kann.
vi) Zusätzlich zu v) kann die Umlenkfläche mindestens eine Rinne, bevorzugt eine Vielzahl von Rinnen aufweisen, welche derart ausgebildet und angeordnet ist/sind, dass das Gut darin rutschen kann.
vii) Zusätzlich zu einem der Merkmale i) bis vi) kann die Vorrichtung stromaufwärts von der ersten Vibrationsfläche eine im Wesentlichen ebene und bevorzugt im Wesentlichen horizontal ausgerichtete zweite Vibrationsfläche aufweisen, welche zu Vibrationen anregbar ist.
viii) Zusätzlich zu einem der Merkmale i) bis vii) kann die Vorrichtung stromabwärts von der Behandlungszone eine Sortiereinrichtung aufweist, welche eine Messeinheit und eine Ausstosseinheit enthält, welche derart ausgebildet sind, dass mittels der Ausstosseinheit einzelne Partikel des Guts anhand mindestens einer mittels der Messeinheit gemessenen Eigenschaft der Partikel ausstossbar sind.
   Die Vorrichtung kann mindestens eine stromabwärts von der Behandlungszone angeordnete Gasaustrittsöffnung zum Einblasen eines Reinigungsgases auf das Gut aufweisen.

Bei dem Gut kann es sich um ein Lebensmittel handeln, wie beispielsweise Getreide wie etwa Soja, Frühstückscerealien, Snacks, Nüsse wie etwa getrocknete Kokosnüsse, Mandeln, Erdnussbutter, Kakaobohnen, Schokolade, Schokoladenflüssigkeit, Schokoladenpulver, Schokoladenchips, Kakaoprodukte, Hülsenfrüchte, Kaffee, Samen wie etwa Kürbissamen, Gewürze (wie beispielsweise Kurkuma, insbesondere in Scheiben), Teemischungen, getrocknete Früchte, Pistazien, trockene Proteinprodukte, Bäckereiprodukte, Zucker, Kartoffelprodukte, Teigwaren, Babynahrung, getrocknete Eiprodukte, Sojaprodukte wie beispielsweise Sojabohnen, Verdickungsmittel, Hefen, Hefeextrakte, Gelatine oder Enzyme handeln.

Alternativ kann das Gut auch ein Tiernahrungsmittel sein, wie beispielsweise Pellets, Futter für Wiederkäuer, Geflügel, Wassertiere (insbesondere Fische) oder Haustiere, oder Mischfutter.

Es ist jedoch ebenso denkbar und liegt im Rahmen der Erfindung, dass das Gut beispielsweise ein Kunststoff wie etwa PET ist, beispielsweise in Form von Flocken oder Pellets.

Die Aufgabe wird ausserdem durch ein Verfahren zum Pasteurisieren und/oder Sterilisieren von partikelförmigem Gut gemäss Anspruch 16 gelöst. Vorteilhafte Verfahren sind in den Ansprüchen 17-18 definiert.

Es wird mittels der Elektronenquellen ein Elektronenstrahl erzeugt. Gut, welches insbesondere frei fällt, wird mittels des Elektronenstrahls in der Behandlungszone pasteurisiert und/oder sterilisiert. Ein Kühlfluid wir durch den Hohlraum des Halterahmens zum Kühlen des Schutzelements geleitet.

Das Kühlfluid kann beim Eintritt in den Hohlraum eine Temperatur im Bereich von 15 °C bis 43 °C haben, bevorzugt von 25°C. Die Temperatur sollte grösser als 15 °C sein, um Kondensation, vor allem auf dem Schutzelement zu vermeiden. Beim Austritt liegt eine Temperatur im Bereich von 30°C bis 50°C, bevorzugt von 36°C vor.

Vorteilhafterweise wird das Kühlfluid mit einem Volumenstrom im Bereich von 2-6 1/min m, bevorzugt 4 1/min, durch den Hohlraum geleitet.

Bei dem Kühlfluid kann es sich insbesondere um Luft, Stickstoff oder Wasser handeln.

Das Verfahren kann zudem eines oder mehrere der Merkmale aufweisen, die in der eingangs bereits erwähnten internationalen Patentanmeldung PCT/EP2017/070842 der Anmelderin offenbart sind, nämlich
a) Fördern und Vereinzeln des Guts mittels einer bevorzugt im Wesentlichen horizontal ausgerichteten ersten Vibrationsfläche, welche zu Vibrationen angeregt wird und eine Vielzahl von Rinnen aufweist, in denen das Gut gefördert und mittels deren es vereinzelt wird.
b) Zusätzlich zu Merkmal a) kann das Gut umgebendes Prozessgas nach der Pasteurisierung und/oder Sterilisierung abgesaugt werden, insbesondere mit einer Absauggeschwindigkeit, die das 1- bis 3-fache, bevorzugt das 1- bis 1,5-fache der Geschwindigkeit des Guts während der Pasteurisierung und/oder Sterilisierung beträgt.

Im Folgenden wird die Erfindung anhand mehrerer Ausführungsbeispiele und mehrerer Zeichnungen näher erläutert. Elemente mit gleicher Funktion haben jeweils die gleichen Bezugszeichen.

Dabei zeigen
- Figur 1:: eine schematische Seitenansicht einer erfindungsgemässen Vorrichtung;
- Figur 2:: eine seitliche Ansicht einer Behandlungszone der erfindungsgemässen Vorrichtung;
- Figur 3:: eine perspektivische geschnittene Detailansicht eines ersten Beispiels für eine Kassette der erfindungsgemässen Vorrichtung;
- Figur 4:: eine perspektivische geschnittene Detailansicht eines zweiten Beispiels für eine Kassette der erfindungsgemässen Vorrichtung;
- Figur 5:: eine erste perspektivische Ansicht eines dritten Beispiels für eine Kassette der erfindungsgemässen Vorrichtung;
- Figur 6:: eine weitere perspektivische Ansicht des dritten Beispiels für eine Kassette der erfindungsgemässen Vorrichtung;
- Figur 7:: eine das dritte Beispiel für eine Kassette der erfindungsgemässen Vorrichtung in einer Schnittansicht.

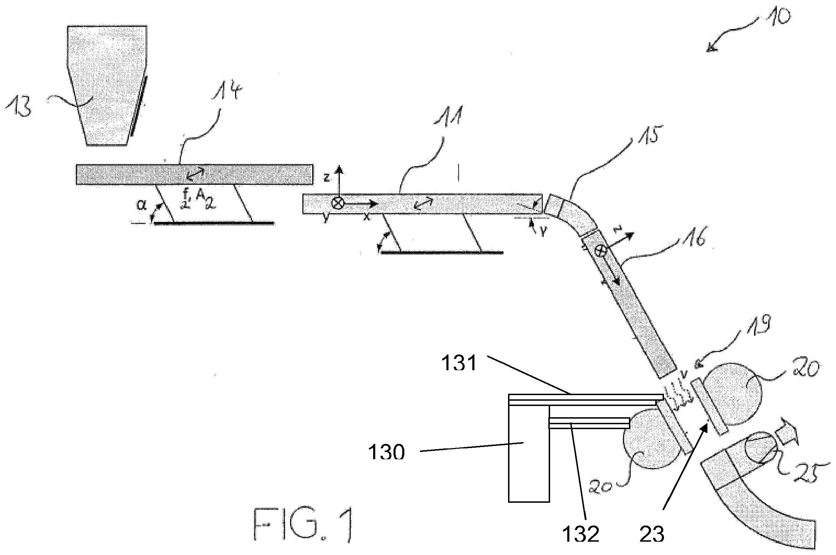

Die in Figur 1 dargestellte Vorrichtung 10 ist zum Pasteurisieren und/oder Sterilisieren von partikelförmigem Gut vorgesehen, wie etwa einem Gewürz, Sesam, Mandeln oder geschälten Pistazien. Sie enthält eine Dosiereinrichtung 13, mit der das Gut auf eine zweite Vibrationsfläche 14 dosiert werden kann. Mit Hilfe dieser zweiten Vibrationsfläche 14 kann der Durchsatz des Guts kontrolliert werden, und es kann auch bereits eine Vorvereinzelung stattfinden.

Stromabwärts von der zweiten Vibrationsfläche 14 enthält die Vorrichtung 10 eine horizontal ausgerichtete erste Vibrationsfläche 11. Hierdurch kann das Gut weiter stromabwärts gefördert und vereinzelt werden.

Stromabwärts von der ersten Vibrationsfläche 11 weist die Vorrichtung 10 eine Umlenkfläche 15 auf. Diese ist derart ausgebildet und angeordnet, dass das Gut darauf umgelenkt und von der ersten Vibrationsfläche 11 zu einer Rutschfläche 16 rutschen kann. Die Umlenkfläche 15 ist derart auf das Gut und die erste Vibrationsfläche 11 abgestimmt, dass die Partikel des Guts auf einer Parabelbahn im Wesentlichen stromabwärts geführt werden, auf der sie auch allein aufgrund der Einwirkung der Schwerkraft fallen würden.

Noch weiter stromabwärts enthält die Vorrichtung 10 eine Behandlungszone 19. Dort wird das Gut frei fallend mittels eines Elektronenstrahls pasteurisiert und/oder sterilisiert, der von zwei einander gegenüberliegenden Elektronenquellen 20 erzeugt wird.

Die Vorrichtung 10 enthält weiterhin eine Absaugeinrichtung 25, mit dem Prozessgas, welches das Gut umgibt, stromabwärts von der Behandlungszone 19 abgesaugt werden kann.

Zum Pasteurisieren und/oder Sterilisieren von partikelförmigem Gut mit Hilfe dieser Vorrichtung 10 werden die folgenden Schritte durchgeführt:
Mittels der zweiten Vibrationsfläche 14 wird der Durchsatz des Guts kontrolliert, und es findet eine Vorvereinzelung statt. Mittels der Elektronenquellen 20 wird in einem weiteren Schritt ein Elektronenstrahl erzeugt. In einem weiteren Schritt erfolgt dann ein Pasteurisieren und/oder Sterilisieren des frei fallenden Guts mittels des Elektronenstrahls in der Behandlungszone 19.

Das Gut bewegt sich im Falle von Gewürzen vorteilhaft mit einer Geschwindigkeit im Bereich von 1 m/s bis 5 m/s, bevorzugt von 2 m/s bis 4 m/s, besonders bevorzugt von 2 m/s bis 3 m/s, zum Beispiel von 2,5 m/s durch die Behandlungszone 19. Diese Geschwindigkeit kann durch die Länge und den Neigungswinkel und die Länge der Rutschfläche 17 eingestellt werden.

Je höher die Geschwindigkeit des Guts ist, desto grösser ist der erreichbare Durchsatz. Beim freien Fall ist die Geschwindigkeit unabhängig vom Durchsatz, so dass beispielsweise Durchsätze im Bereich 100 kg/h bis 1000 kg/h bei der gleichen Geschwindigkeit erreicht werden können.

Der Durchsatz kann von der Vibration einer Vibrationsflächen 11, 14 und der Dimensionen und Orientierungen der Umlenkfläche 15 und der Rutschfläche 16 abhängen. Zudem sinkt mit steigender Geschwindigkeit des Guts die Wahrscheinlichkeit von Kollisionen der Partikel mit der Elektronenquelle 20 oder eines Schutzelements 23. Andererseits dürfen die Geschwindigkeiten aber auch nicht zu gross gewählt werden, damit das Gut ausreichend lange im Elektronenstrahl verbleibt, um pasteurisiert und/oder sterilisiert zu werden.

Die Elektronen des Elektronenstrahls weisen eine Energie auf, die im Bereich von 80 keV bis 300 keV, bevorzugt von 140 keV bis 280 keV, besonders bevorzugt von 180 keV bis 260 keV liegt, beispielsweise bei 250 keV. Geringere Elektronenenergien würden keine ausreichende Pasteurisierung und/oder Sterilisierung erzeugen. Durch höhere Elektronenenergien liessen sich keine wesentlich höheren Grade der Pasteurisierung und/oder Sterilisierung erreichen.

In der Behandlungszone 19 weist der Elektronenstrahl eine mittlere Stromdichte auf, die im Bereich von 10¹⁵ s⁻¹·cm⁻² bis 2,77·10¹⁵ s⁻¹·cm⁻² liegt. Das Gut wird dem Elektronenstrahl für eine Behandlungszeit ausgesetzt, die im Bereich von 5 ms bis 25 ms liegen und beispielsweise 15 ms betragen kann. Denn für eine ausreichende Pasteurisierung und/oder Sterilisierung ist eine gewisse minimale Behandlungszeit nötig. Zu lange Behandlungszeiten haben keinen nennenswert erhöhten Grad der Pasteurisierung und/oder Sterilisierung gezeigt und würden zudem den Durchsatz verringern.

Hierdurch wird das Gut einer Strahlendosis ausgesetzt, die im Bereich von 1 kGy bis 45 kGy liegen, bevorzugt von 8 kGy bis 30kGy, besonders bevorzugt von 10 kGy bis 16 kGy, und beispielsweise 12 kGy betragen kann.

Das das Gut umgebende Prozessgas wird nach der Pasteurisierung und/oder Sterilisierung in der Behandlungszone 19 mittels der Absaugeinrichtung 25 abgesaugt, und zwar mit einer bevorzugten Absauggeschwindigkeit, die das 1- bis 1,5-fache Geschwindigkeit des Guts während der Pasteurisierung und/oder Sterilisierung beträgt.

Die Vorrichtung 10 umfasst ausserdem eine Kühlvorrichtung 130, welche zwei Kühlkreisläufe versorgt. Ein Kühlkreislauf 132 führt Kühlfluid der Elektronenquelle 20 zu, eine weiterer Kühlkreislauf 131 führt Kühlfluid dem Schutzelement 23 zu, welches die Behandlungszone 19 von den Elektronenquellen 20 trennt.

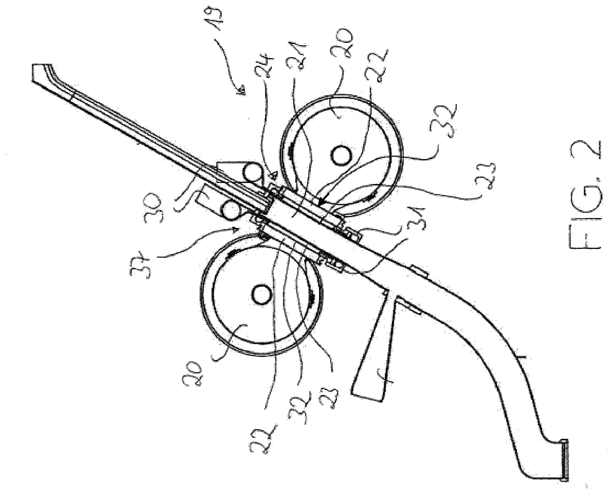

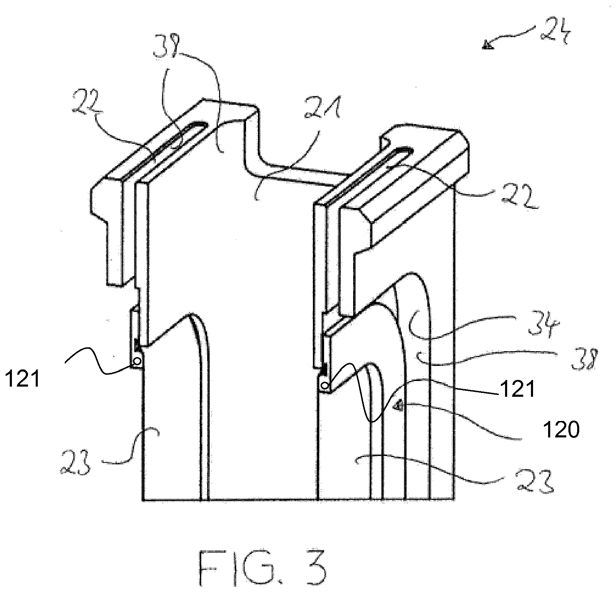

In Figur 2 ist die Behandlungszone 19 in einer Detailansicht dargestellt. Im Bereich der Behandlungszone 19 weist die Vorrichtung 10 eine zwischen Austrittsfenstern 32 der Elektronenquellen 20 angeordnete Kassette 24 auf. Die Kassette 24 ist in einer Kassettenaufnahme 37 eingesetzt. Die Kassette 24 enthält zwei Halterahmen 120 (siehe Figur 3) für jeweils eine aus Titan bestehendes Schutzelement 23, das für die Elektronenstrahlen teilweise durchlässig ist. Die Kassette 24 enthält mehrere Begrenzungsflächen 38 (siehe Figur 3), die zusammen mit den Schutzfolien 23 einen Gutkanal 21 begrenzen, in dem das Gut mittels der Elektronenstrahlen pasteurisierbar und/oder sterilisierbar ist.

Weiterhin enthält die Vorrichtung 10 im Bereich der Behandlungszone 19 zwei Nebenkanäle 22. Diese werden in einer Betriebsposition durch in der in Figur 3 gezeigte Begrenzungsflächen 38 der Kassette 24, die Schutzfolie 23 und die Austrittsfenster 32 der Elektronenquellen 20 begrenzt und verlaufen somit zwischen dem Gutkanal 21 und den Elektronenquellen 20.

Der Gutkanal 21 ist von den Nebenkanälen 23 fluidgetrennt, und zwar unter anderem durch das Schutzelement.

Durch Eintrittsöffnungen 30 kann Luft eingeführt werden, die die Nebenkanäle 23 parallel zur Strömungsrichtung des Guts durchströmen kann. Stromabwärts kann die Luft aus Austrittsöffnungen 31 wieder austreten. Dieser Luftstrom ermöglicht einerseits das Abführen von Ozon, welches durch die Elektronenstrahlen erzeugt wird, und andererseits eine Kühlung der Elektronenquellen 20 und insbesondere ihrer Austrittsfenster 32.

In Figur 3 ist eine noch detaillierte, geschnittene und perspektivische Ansicht eines ersten Beispiels einer Kassette 24 gezeigt, in der der Gutkanal 21, die beiden Nebenkanäle 22 und die beiden Schutzelemente 23 erkennbar sind.

Der Gutkanal 21 ist durch die beiden Schutzelemente 23 von den Nebenkanälen 22 fluidgetrennt.

Die Schutzelemente 23 sind in einem Halterahmen 120 eingespannt.

An den dem Gutkanal 21 abgewandten Seiten der Kassette 24 ist jeweils eine Aussparung 34 gebildet, die in der Betriebsposition der Vorrichtung 10 von Austrittsfenstern 32 der Elektronenquellen 20 verschlossen werden und durch die die Elektronenstrahlen hindurchdringen können.

Der Halterahmen weist einen von einem Kühlfluid durchströmbaren Hohlraum 121 auf. Der Hohlraum 121 liegt vollständig innerhalb des Halterahmens 120.

Der Hohlraum 121 ist Teil eines separaten Kühlkreislaufs 131 (siehe Figur 1), welcher von einem Kühlfluid durchströmt wird. Der Kühlkreislauf 131 kann zu derselben Kühlvorrichtung 130 gehören, die auch die Elektronenquelle 20 kühlt (siehe Figur 1) .

Der Hohlraum 121 kann beispielsweise flüssiges Kühlfluid führen, während die Nebenkanäle 22 von Gas durchströmt werden.

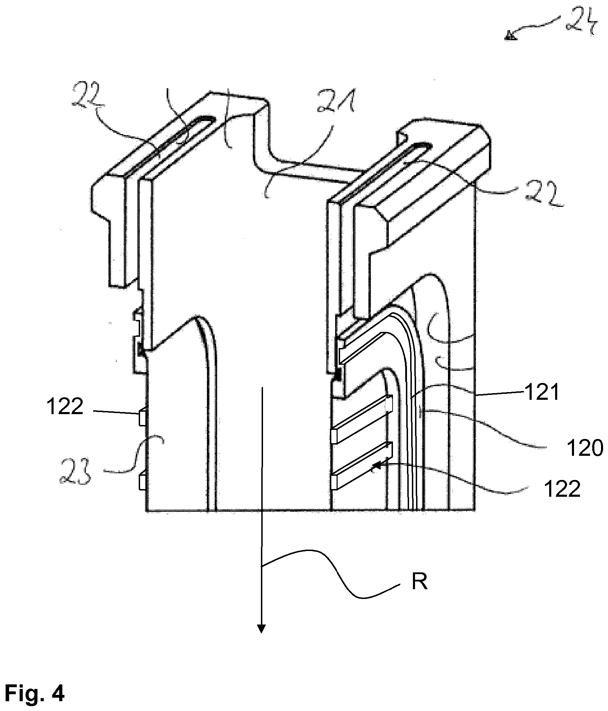

In Figur 4 zeigt eine perspektivische Ansicht eines zweiten Beispiels einer Kassette 24, in der ebenfalls der Gutkanal 21, die beiden Nebenkanäle 22 und die beiden Schutzelemente 23 erkennbar sind.

Die Hohlräume sind zu den Nebenkanälen 22 offen. Kühlfluid, welches die Nebenkanäle durchströmt, kühlt somit besonders effektiv den Halterahmen 120. Die Nebenkanäle 22 und die Hohlräume 121 können Teil eines Kühlkreislaufs 131 (siehe Figur 1) sein.

In diesem Beispiel enthält das Schutzelement 23 mehrere Verdickungen 122, die sich entlang einer Hauptebene des Schutzelements 23 und im Wesentlichen senkrecht zu einer Gutströmungsrichtung R erstrecken. Die Verdickungen 122 stabilisieren das Schutzelement 23.

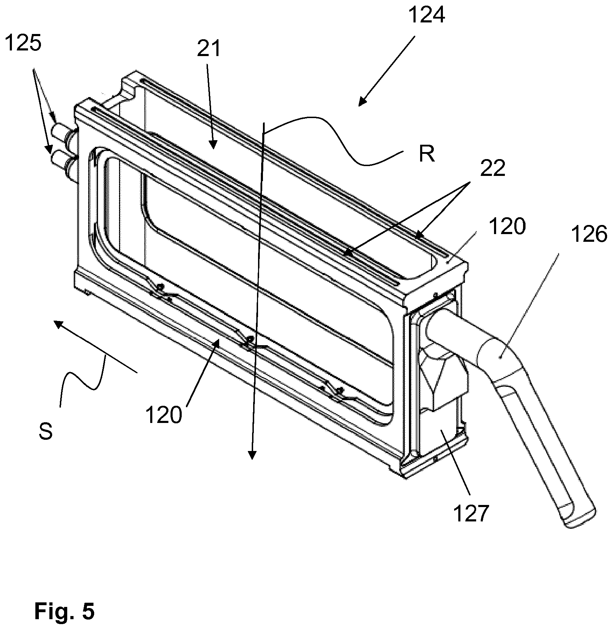

Figur 5 zeigt eine erste perspektivische Ansicht eines dritten Beispiels für eine Kassette 124 der erfindungsgemässen Vorrichtung 10.

Die Kassette 124 ist in eine Richtung S quer zur Gutströmungsrichtung R in eine nicht explizit dargestellte Kassettenaufnahme der Vorrichtung 10 (siehe Figur 1) einschiebbar und weist dazu einen Griff 126 auf, der an einer Griffplatte 127 angebracht ist.

In der Kassette 124 sind der Gutkanal 21 und zwei Nebenkanäle ausgebildet.

Die Kassette umfasst als integrale Bestandteile ausserdem zwei Halterahmen 120 zum Halten von zwei nicht explizit dargestellten Schutzelementen.

Die Kassette 124 und damit die Halterahmen 120 weisen einen Hohlraum 121 (siehe Figur 2) zum Durchleiten einer Kühlflüssigkeit auf. Der Hohlraum 121 ist in Form einer durchgehenden Röhre ausbildet und schliesst jeweils an einen Stutzen 125 an zum Einleiten und Ausleiten der Flüssigkeit. Die Stutzen 125 sind als Fluidstecker ausgebildet, welche die Verbindung zu einem Kühlkreislauf 131 (siehe Figur 1) herstellen können.

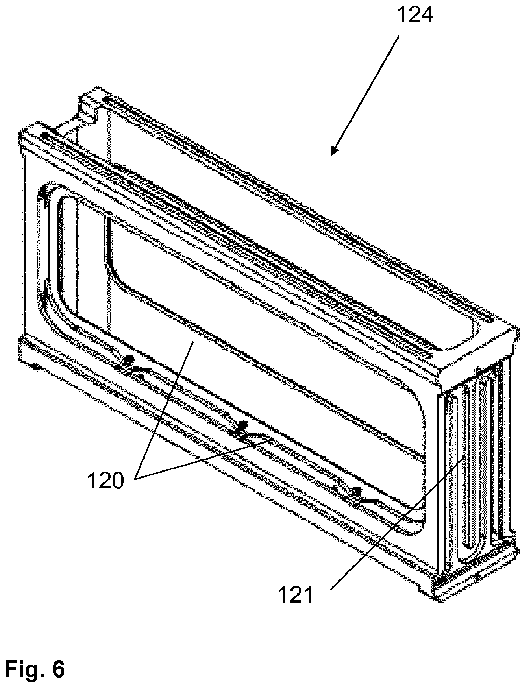

Figur 6 zeigt die gleiche perspektivische Ansicht des dritten Beispiels für eine Kassette 124 der erfindungsgemässen Vorrichtung. In dieser Ansicht ist die Griffplatte 127 (siehe Figur 5) nicht dargestellt. Dadurch ist der Hohlraum 121 sichtbar. Dieser mäandert als Nut durch die Kassette 124, welche gleichzeitig die Halterahmen 120 für die nicht explizit dargestellten Schutzelemente bildet.

Nach Aufschrauben oder Aufschweissen der Griffplatte 127 (siehe Figur 5) ist der Hohlraum 121 eine geschlossene Röhre.

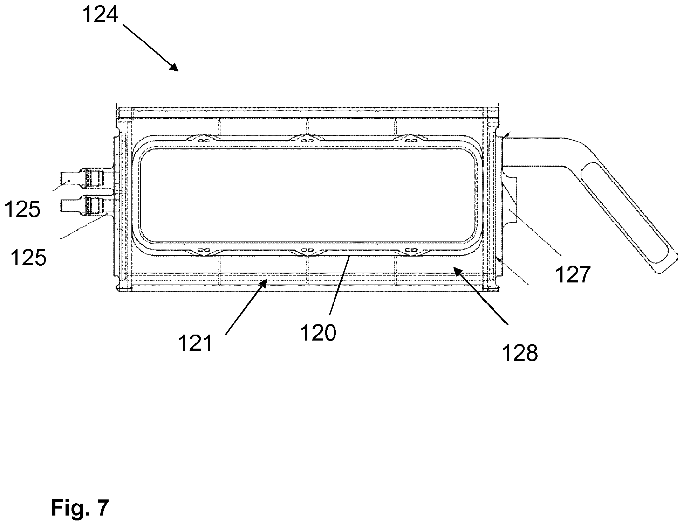

Figur 7 zeigt das dritte Beispiel für eine Kassette 124 der erfindungsgemässen Vorrichtung in einer Schnittansicht.

Der Hohlraum 121 führt als geschlossene Röhre von den Stutzen 125 durch den unteren Bereich 128 der Kassette 124, an der Griffplatte 127 vorbei und wieder zurück. Auf dem Weg zwischen Stutzen 125 und Griffplatte 127 wird der erste Halterahmen 120 gekühlt, auf dem Weg von der Griffplatte 127 zurück zu den Stutzen 125 verläuft der Hohlraum 121 durch den zweiten Halterahmen 120.

## Patentansprüche

1. Vorrichtung (10) zum Pasteurisieren und/oder Sterilisieren von partikelförmigem Gut, enthaltend
- mindestens zwei Elektronenquellen (20) zum Erzeugen eines Elektronenstrahls, die einander gegenüberliegen,
- eine Behandlungszone (19), in der das Gut frei fallend mittels des Elektronenstrahls pasteurisierbar und/oder sterilisierbar ist,
- einen im Bereich der Behandlungszone (19) angeordneten Gutkanal (21), in dem das Gut mittels des Elektronenstrahls pasteurisierbar und/oder sterilisierbar ist,
- ein zwischen der Elektronenquelle (20) und dem Gutkanal (21) angeordnetes, für den Elektronenstrahl zumindest teilweise durchlässiges und insbesondere aus einem Metall, bevorzugt aus Titan, bestehendes, flächiges Schutzelement (23),
**dadurch gekennzeichnet, dass**
die Vorrichtung (10) eine Kassettenaufnahme (37) und eine Kassette (24) mit Halterahmen (120) umfasst, wobei die Kassette (24) zwei Halterahmen (120) für zwei gegenüber liegende Schutzelemente (23) umfasst, wobei die Halterahmen (120) die Schutzelemente (23) halten und einen von einem Kühlfluid durchströmbaren Hohlraum (121) aufweisen.

2. Vorrichtung (10) Anspruch 1,
wobei der Hohlraum (121) als geschlossene Röhre ausgebildet ist, bevorzugt mit einem Durchmesser von 3-8mm.

3. Vorrichtung (10) gemäss einem der vorangehenden Ansprüche,
wobei die Schutzelemente (23) als Schutzfolie (23) ausgebildet sind.

4. Vorrichtung (10) gemäss einem der vorangehenden Ansprüche,
wobei die Vorrichtung (10) mindestens einen von einem Fluid durchströmbaren Nebenkanal (22) aufweist, welcher zumindest teilweise zwischen Elektronenquelle (20) und Gutkanal (21) verläuft und vom Gutkanal (21) fluidgetrennt ist.

5. Vorrichtung (10) gemäss Anspruch 4,
wobei das Schutzelement (23) den Gutkanal (21) vom Nebenkanal (22) trennt.

6. Vorrichtung (10) gemäss einem der Ansprüche 4 oder 5, wobei der Nebenkanal (22) zumindest teilweise zwischen Elektronenquelle (20) und Schutzelement (23) angeordnet ist.

7. Vorrichtung (10) gemäss einem der vorangehenden Ansprüche,
wobei die Schutzelemente (23), insbesondere die Schutzfolien (23), mindestens eine, bevorzugt mehrere Verdickungen (122) enthalten, die sich entlang einer Hauptebene des Schutzelements (23) und im Wesentlichen senkrecht zu einer Gutströmungsrichtung (R) erstrecken.

8. Vorrichtung (10) gemäss einem der vorangehenden Ansprüche,
wobei die Vorrichtung (10) eine Kühlvorrichtung (130) mit zwei miteinander verbundenen Kühlkreisläufen (131, 132) enthält, wobei ein Kühlfluid mittels eines ersten Kühlkreislaufs (131) dem Halterahmen (120) zuführbar ist und mittels eines zweiten Kühlkreislaufs (132) der Elektronenquelle (20) zuführbar ist.

9. Vorrichtung (10) gemäss einem der vorangehenden Ansprüche,
wobei die Vorrichtung (10) mindestens ein auf das Schutzelement (23) gerichtetes Gebläse zur weiteren Kühlung des Schutzelements (23) enthält.

10. Vorrichtung (10) gemäss einem der vorangehenden Ansprüche,
wobei die Halterahmen (120) integrierter Bestandteil der Kassette sind.

11. Vorrichtung gemäss Anspruch 1, wobei die Kassette den kompletten Gutkanal enthält.

12. Vorrichtung gemäss Anspruch 1 oder 11, wobei die Halterahmen lösbar in der Kassette aufgenommen oder aufnehmbar sind.

13. Vorrichtung gemäss Anspruch 1, 10, 11 oder 12, wobei in der Kassette eine Druckmessvorrichtung angeordnet ist.

14. Vorrichtung gemäss einem der Ansprüche 1 und 10 bis 14, wobei die Elektronenquellen derart beweglich, insbesondere schwenkbar und/oder verschiebbar, relativ zur Kassettenaufnahme angeordnet sind, dass sie von der Kassette weg bewegbar sind.

15. Vorrichtung gemäss einem der Ansprüche 1, 10 bis 13, wobei der Hohlraum in der Kassette als durchgehende Röhre ausgebildet ist und zur Kühlung beider Schutzelemente und der Kassette dient.

16. Verfahren zum Pasteurisieren und/oder Sterilisieren von partikelförmigem Gut mit einer Vorrichtung (10) gemäss einem der vorangehenden Ansprüche, enthaltend die folgenden Schritte:
a) Erzeugen eines Elektronenstrahls mittels der Elektronenquellen (20),
b) Pasteurisieren und/oder Sterilisieren des insbesondere frei fallenden Guts mittels des Elektronenstrahls in der Behandlungszone (19),
c) Durchleiten eines Kühlfluids durch den Hohlraum (121) der Halterahmenen (120) zum Kühlen des Schutzelements (23).

17. Verfahren gemäss Anspruch 16,
wobei das Kühlfluid beim Eintritt in den Hohlraum (121) eine Temperatur im Bereich von 15°C bis 43°C hat, bevorzugt von 25°C, hat.

18. Verfahren gemäss einem der Ansprüche 16 und 17,
wobei das Kühlfluid mit einem Volumenstrom im Bereich von 31/min bis 51/min durch den Hohlraum (121) geleitet wird.

## Claims

1. Apparatus (10) for pasteurizing and/or sterilizing particulate material, comprising
- at least two electron sources (20) for generating an electron beam, which are located opposite one another,
- a treatment zone (19) in which the material can be pasteurized and/or sterilized in free-falling by means of the electron beam,
- a material channel (21) arranged in the region of the treatment zone (19), in which the material can be pasteurized and/or sterilized by means of the electron beam,
- a flat protective element (23) arranged between the electron source (20) and the material channel (21), at least partially permeable to the electron beam and consisting in particular of a metal, preferably titanium,
**characterized in that**
the device (10) comprises a cassette holder (37) and a cassette (24) with holding frames (120), the cassette (24) comprising two holding frames (120) for two opposing protective elements (23), the holding frames (120) holding the protective elements (23) and having a cavity (121) through which a cooling fluid can flow.

2. Device (10) claim 1,
wherein the cavity (121) is formed as a closed tube, preferably with a diameter of 3-8mm.

3. Device (10) according to one of the preceding claims,
wherein the protective elements (23) are formed as a protective film (23).

4. Device (10) according to one of the preceding claims,
wherein the device (10) has at least one secondary channel (22) through which a fluid can flow, which extends at least partially between the electron source (20) and the material channel (21) and is fluid-separated from the material channel (21).

5. Device (10) according to claim 4, wherein the protective element (23) separates the material channel (21) from the secondary channel (22).

6. Device (10) according to one of claims 4 or 5, wherein the secondary channel (22) is arranged at least partially between the electron source (20) and the protective element (23).

7. Device (10) according to one of the preceding claims,
wherein the protective elements (23), in particular the protective foils (23), comprise at least one, preferably several, thickenings (122) extending along a main plane of the protective element (23) and substantially perpendicular to a material flow direction (R).

8. Device (10) according to any one of the preceding claims, wherein the device (10) comprises a cooling device (130) with two interconnected cooling circuits (131, 132), wherein a cooling fluid can be supplied to the holding frame (120) by means of a first cooling circuit (131) and can be supplied to the electron source (20) by means of a second cooling circuit (132).

9. Device (10) according to one of the preceding claims,
wherein the device (10) contains at least one fan directed at the protective element (23) for further cooling of the protective element (23).

10. Device (10) according to one of the preceding claims,
wherein the holding frames (120) are an integral part of the cassette.

11. Device according to claim 1, wherein the cassette contains the complete material channel.

12. Device according to claim 1 or 11, wherein the holding frames are detachably accommodated or removable in the cassette.

13. Device according to claim 1, 10, 11 or 12, wherein a pressure measuring device is arranged in the cassette.

14. Device according to one of claims 1 and 10 to 14,
wherein the electron sources are arranged movably, in particular pivotably and/or displaceably, relative to the cassette receptacle in such a way that they can be moved away from the cassette.

15. Device according to one of claims 1, 10 to 13, wherein the cavity in the cassette is formed as a continuous tube and serves for cooling both protective elements and the cassette.

16. Method for pasteurizing and/or sterilizing particulate material with an apparatus (10) according to any one of the preceding claims, comprising the following steps:
a) generating an electron beam by means of the electron sources (20),
b) pasteurizing and/or sterilizing the material, in particular free-falling material, by means of the electron beam in the treatment zone (19),
c) passing a cooling fluid through the cavity (121) of the holding frames (120) for cooling the protective element (23).

17. Method according to claim 16, wherein the cooling fluid on entering the cavity (121) has a temperature in the range of 15°C to 43°C, preferably 25°C.

18. Method according to any one of claims 16 and 17, wherein the cooling fluid is passed through the cavity (121) at a flow rate in the range of 31/min to 51/min.

## Revendications

1. Dispositif (10) pour la pasteurisation et/ou la stérilisation de produits en forme de particules, comprenant
- au moins deux sources d'électrons (20) pour la production d'un faisceau d'électrons, qui se font face,
- une zone de traitement (19) dans laquelle la matière peut être pasteurisée et/ou stérilisée en chute libre au moyen du faisceau d'électrons,
- un canal de produits (21) disposé dans la zone de traitement (19), dans lequel les produits peuvent être pasteurisés et/ou stérilisés au moyen du faisceau d'électrons,
- un élément de protection (23) plat, disposé entre la source d'électrons (20) et le canal de produit (21), au moins partiellement perméable au faisceau d'électrons et constitué en particulier d'un métal, de préférence du titane,
**caractérisé en ce que**
le dispositif (10) comprend un logement de cassette (37) et une cassette (24) avec des cadres de maintien (120), la cassette (24) comprenant deux cadres de maintien (120) pour deux éléments de protection (23) opposés, les cadres de maintien (120) maintenant les éléments de protection (23) et présentant une cavité (121) pouvant être traversée par un fluide de refroidissement.

2. Dispositif (10) revendication 1, dans lequel la cavité (121) étant conçue comme un tube fermé, de préférence d'un diamètre de 3 à 8 mm.

3. Dispositif (10) selon l'une quelconque des revendications précédentes, dans lequel les éléments de protection (23) sont conçus comme un film de protection (23).

4. Dispositif (10) selon l'une des revendications précédentes, dans lequel le dispositif (10) présente au moins un canal secondaire (22) pouvant être traversé par un fluide, qui s'étend au moins partiellement entre la source d'électrons (20) et le canal de produit (21) et qui est séparé du canal de produit (21) par un fluide.

5. Dispositif (10) selon la revendication 4, dans lequel l'élément de protection (23) séparant le canal de produit (21) du canal secondaire (22).

6. Dispositif (10) selon l'une des revendications 4 ou 5, dans lequel le canal secondaire (22) est disposé au moins partiellement entre la source d'électrons (20) et l'élément de protection (23).

7. Dispositif (10) selon l'une des revendications précédentes, dans lequel les éléments de protection (23), en particulier les films de protection (23), contiennent au moins une, de préférence plusieurs surépaisseurs (122), qui s'étendent le long d'un plan principal de l'élément de protection (23) et sensiblement perpendiculairement à une direction d'écoulement de la matière (R).

8. Dispositif (10) selon l'une des revendications précédentes, dans lequel le dispositif (10) comprend un dispositif de refroidissement (130) avec deux circuits de refroidissement (131, 132) reliés entre eux, un fluide de refroidissement pouvant être amené au cadre de maintien (120) au moyen d'un premier circuit de refroidissement (131) et à la source d'électrons (20) au moyen d'un deuxième circuit de refroidissement (132).

9. Dispositif (10) selon l'une des revendications précédentes, dans lequel le dispositif (10) comprend au moins une soufflerie dirigée vers l'élément de protection (23) pour refroidir davantage l'élément de protection (23).

10. Dispositif (10) selon l'une des revendications précédentes, dans lequel les cadres de maintien (120) font partie intégrante de la cassette.

11. Dispositif selon la revendication 1, dans lequel la cassette contient le canal à produits complet.

12. Dispositif selon la revendication 1 ou 11, dans lequel les cadres de maintien sont logés ou peuvent être logés de manière amovible dans la cassette.

13. Dispositif selon la revendication 1, 10, 11 ou 12, dans lequel un dispositif de mesure de pression est disposé dans la cassette.

14. Dispositif selon l'une des revendications 1 et 10 à 14, dans lequel les sources d'électrons sont disposées de manière mobile, en particulier de manière pivotante et/ou coulissante, par rapport au logement de la cassette, de sorte qu'elles peuvent être éloignées de la cassette.

15. Dispositif selon l'une des revendications 1, 10 à 13, dans lequel la cavité dans la cassette est conçue comme un tube continu et sert à refroidir les deux éléments de protection et la cassette.

16. Procédé de pasteurisation et/ou de stérilisation de produits particulaires avec un dispositif (10) selon l'une des revendications précédentes, comprenant les étapes suivantes :
a) Génération d'un faisceau d'électrons au moyen des sources d'électrons (20),
b) Pasteurisation et/ou stérilisation de la matière, en particulier en chute libre, au moyen du faisceau d'électrons dans la zone de traitement (19),
c) passage d'un fluide de refroidissement à travers la cavité (121) des cadres de maintien (120) pour refroidir l'élément de protection (23).

17. Procédé selon la revendication 16, dans lequel le fluide de refroidissement a une température comprise entre 15°C et 43°C, de préférence 25°C, lorsqu'il pénètre dans la cavité (121) .

18. Procédé selon l'une des revendications 16 et 17, dans lequel le fluide de refroidissement est envoyé à travers la cavité (121) avec un débit volumique compris entre 31/min et 51/min.
